Europäisches Patentamt

European Patent Office

Office européen des brevets

(11) Publication number: **0 147 517 B1**

# EUROPEAN PATENT SPECIFICATION

(45) Date of publication of patent specification: **29.04.92** (51) Int. Cl.⁵: **C12N 1/00, C12P 1/00**

(21) Application number: **84108564.0**

(22) Date of filing: **19.07.84**

The file contains technical information submitted after the application was filed and not included in this specification

(54) Method of removing malodorous or unpleasant taste imparting substances from microbial cells or fermentation liquors.

(30) Priority: **26.12.83 JP 247905/83**

(43) Date of publication of application:
**10.07.85 Bulletin  85/28**

(45) Publication of the grant of the patent:
**29.04.92 Bulletin  92/18**

(84) Designated Contracting States:
**DE FR GB IT SE**

(56) References cited:
| | |
|---|---|
| EP-A- 0 034 872 | CH-A- 62 577 |
| CH-A- 537 707 | DE-C- 941 461 |
| FR-A- 1 560 717 | FR-A- 2 273 868 |
| US-A- 2 071 346 | US-A- 4 166 135 |

CHEMICAL ABSTRACTS, vol. 87, no. 11, 12th September 1977, page 469, abstract no. 83380u, Columbus, Ohio, US; & JP-A-77 61 257 (KIKKOMAN SHOYU CO. LTD) 20-05-1977

CHEMICAL ABSTRACTS, vol. 83, no. 10, 10th November 1975, page 398, abstract no. 162214p, Columbus, Ohio, US; & JP-A-75 70 578 (MITSUBISHI GAS CHEMICAL CO., INC.)

12-06-1975

(73) Proprietor: **Chugai Seiyaku Kabushiki Kaisha**
**5-1, 5-chome, Ukima Kita-ku**
**Tokyo(JP)**

(72) Inventor: **Yotsuhashi, Kazuhiro**
**1-1019, Nogikuno**
**Matsudo-shi Chiba-ken(JP)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 147 517 B1

## Description

The present invention relates to a method of eliminating or reducing malodorous or unpleasant taste imparting substances which are produced during microbial fermentation as employed in food industries but which are difficult to remove by conventional unit operations. More particularly, the invention relates to a method which comprises blowing pressurized steam into flowing fermented whey containing the malodorous or unpleasant taste imparting substances produced during fermentation, thereby bringing the steam into rapid and strong contact with the microbial cells in the fermented whey so that the malodorous or unpleasant taste imparting substances on the microbial cells become easily removable, and subsequently distilling off such substances in vacuum together with the steam.

From the viewpoint of efficient utilization of resources, it is desired to produce highly nutritious and flavorful foods by fermenting by-products resulting from food processing. Microorganisms used in food industries are characterized by strict requirements for nutrients on culture media. For example, the productivity of fermented foods [e.g. soybean sauce, bean paste, natto (sticky fermented whole soybeans), edible vinegar, cheese and yogurt] and alcoholic beverages (e.g. wine, Japanese sake and beer) is reduced if the culture medium does not have a balanced combination of vitamins and minerals or if the medium is deficient in growth factors. Even if the productivity is high, the desired flavors and fragrances are not produced, and instead, malodorous or unpleasant taste imparting substances are formed to greatly impair the value of the final product.

If a medium having a nutritive balance unfavorable to a certain microorganism, for example, a medium made of usually discarded by-products from food processing, is employed as a fermentation medium for the production of valuable foods, microbial strains capable of efficient fermentation to yield highly flavorful and fragrant products must be screened, or alternatively, a technique capable of eliminating or reducing the malodorous or unpleasant taste imparting substances that accompany fermentation must be developed.

However, screening microbial strains capable of efficient fermentation to yield highly flavorful and fragrant products in spite of the poor balance of components in the medium requires tremendous time, labor and cost, and at the same time, the chance of success of this approach is very low.

Instead of this screening method, fermentation may be performed with microbial strains that feature high productivity but which yield less flavorful and fragrant products; in this latter case, any malodor or unpleasant taste must be eliminated from the fermentation liquor, or they must at least be reduced to substantially low levels. As a matter of fact, however, no practical method has been developed that is capable of eliminating or reducing only the malodorous or unpleasant taste imparting substances from the fermentation liquor without damaging any of the useful substances such as protein and vitamins.

Most of the malodorous or unpleasant taste imparting substances that are produced during microbial fermentation in food processing consist of either the extracellular secretion that occurs during the process of assimilation and metabolism of microorganisms, or the biological product of exoenzymes. While typical malodorous or unpleasant taste imparting substances are aldehydes, ketones, alcohols, carboxylic acids and esters thereof, amino acids and peptides, the composition and character of the specific malodor or unpleasant taste varies with the microbial strain, medium and other fermentation conditions.

Those malodorous or unpleasant taste imparting substances which are simply dissolved in the fermentation liquor can be removed fairly easily or reduced to satisfactory levels by conventional engineering techniques, such as adsorption, filtration, extraction, distillation, ion exchange and combinations of these unit operations. However, these unit operations are practically ineffectual for removing or reducing sufficiently the substances that adhere or are adsorbed on the microbial cells.

For example, whey, which is a by-product from the manufacture of cheese or casein, is a good medium for efficient fermentation with a lactic acid bacterium. However, this fermentation produces unbearable types of malodor such as those of cattle pen, wax and sweat, and the fermentation liquor is hardly edible even after it is masked with flavor or seasoning.

This fermentation liquor may be microfiltered to produce a substantially cell-free filtrate which contains a fairly small amount of malodorous or unpleasant taste imparting substances. Most of these substances can be eliminated by concentrating the liquor in vacuum under conditions that avoid protein denaturation or vitamin decomposition. However, the malodorous or unpleasant taste imparting substances cannot be fully removed if a suspension of the microbial cells retained on the filter or the fermentation liquor per se is subjected to concentration under vacuum. Sufficient reduction of the malodorous or unpleasant taste imparting substances may be accomplished by the high-temperature long-time treatment, but then this method causes protein denaturation or vitamin decomposition, and provides a new malodor problem, i.e., "cooking smell".

If microbial cells extracted from the fermentation liquor are washed repeatedly with a solvent such as ethanol, the malodorous or unpleasant taste imparting substances on the cells can be removed fairly easily, but then the smell of the solvent remains, and the use of the solvent is inherently uneconomical.

The present inventors have made various studies to solve these problems involved in using by-products from food processing as a fermentation medium for manufacturing edible products. As a result, the inventors have found that malodorous or unpleasant taste imparting substances on microbial cells in fermented whey can be removed or reduced, without causing such side reactions as protein denaturation or vitamin decomposition by first blowing pressurized steam into the fermented whey and then by concentrating the resulting mixture under vacuum. The present invention has been accomplished on the basis of this finding.

According to the present invention, while the fermented whey is passing through a vessel or pipe capable of withstanding high temperatures and pressures, pressurized steam is blown into the whey thereby bringing the water vapor particles into rapid and strong contact with the surface of the microbial cells in the fermented whey so that the malodorous or unpleasant taste imparting substances on the microbial cells become easily removable, and immediately thereafter, the whey is introduced into a vacuum atmosphere for performing its concentration. During this concentration step, the whey temperature is reduced by the latent heat of evaporation to a sufficiently low level to avoid protein denaturation and vitamin decomposition. At the same time, the malodorous or unpleasant taste imparting substances on the microbial cells, which are usually difficult to remove by conventional unit operations, can be eliminated or decreased to satisfactory levels.

Optimum values of the pressure and temperature of the pressurized steam vary with the proportions and types of the malodorous or unpleasant taste imparting substances adhering to the surface of microbial cells present in the fermented whey to be treated. The requirements to be met are that the target substances should be sufficiently desorbed and that the temperature should not be so high as to cause baking. Therefore, the preferred pressure and temperature ranges are 1 - 10 kg/cm$^2$ (980,6 - 9806 mbar) and 110 - 180°C, respectively. Particularly preferred ranges are 2 - 7 kg/cm$^2$ (1961 - 6860) and 120 - 160°C.

The time interval between the blowing of pressurized steam and the entrance of its mixture with the fermented whey into the vacuum atmosphere should be such that the malodorous or unpleasant taste imparting substances are desorbed from the surface of microbial cells and will not be adsorbed again on the cells, and that neither protein denaturation nor vitamin decomposition occurs. In consideration of these requirements, as well as the pressure of the pressurized steam, the interval between the blowing of pressurized steam and the entrance of its mixture with the fermented whey into the vacuum atmosphere is generally in the range of 1 - 20 seconds, preferably 2 - 10 seconds.

The pressure in the vacuum atmosphere varies with the type and proportions of the malodorous or unpleasant taste imparting substances adhering to the microbial cells in the fermented whey to be treated. In order to ensure high efficiency in the distilling off of the malodorous or unpleasant taste imparting substances and in preventing the loss of useful components by means of the latent heat of evaporation, the pressure in the vacuum atmosphere is preferably as low as possible. But if the pressure is too low, useful components in the fermented whey may be lost together with the foaming materials present in the whey or bubbles evolved in it. Furthermore, creating a high degree of vacuum is difficult and costly. Therefore, in consideration of the pressure of the pressurized steam and the period of treatment, the vacuum atmosphere into which the mixture of fermented whey and pressurized steam is introduced is generally in the range of 40 - 971 mbar preferably 66,5 - 931 mbar, and more preferably 199,5 - 465,5 mbar.

The method of the present invention is suitable for treatment of the whey produced by the fermentation with lactic-acid-producing bacteria or lactic acid bacteria. However, it should be understood that the method can extensively be used to treat other fermentation liquors containing malodorous or unpleasant taste imparting substances, such as fermented foods (e.g. soybean sauce, bean paste, natto, edible vinegar, cheese and yogurt), as well as alcoholic beverages (e.g. wine, Japanese sake and beer).

The method of the present invention is hereunder described in greater detail by means of a reference example and working examples, to which the scope of the present invention is by no means limited.

Reference Example

Microorganisms of the genera Lactobacillus and Streptococcus were grown on media (components: whey or deproteinized whey) until the fermented whey had a final pH of 4.0 and developed an extreme malodor or unpleasant taste. Microbial cells were separated from each of the fermented whey samples by high-speed centrifugation and microfiltration (0.45 μm). The liquid portion had less malodor and unpleasant taste, indicating that most of the malodorous or unpleasant taste imparting substances adhered to the solids portion including the microbial cells. The cells ($10^5$ - $10^6$/ml) were suspended in physiological saline that had been adjusted to pH 4.0 with lactic acid. Each of the suspension samples held at 30°C was heated indirectly (by passage through a plate-type heat exchanger) or directly (by blowing of pressurized steam) to elevate the temperature rapidly to 110°C, 130°C or 150°C. In five seconds, the treated samples were directed into a vacuum atmosphere where they were concentrated at pressures of 199,5 - 332,5 mbar. The resulting products were subjected to an organoleptic test in order to check how much the malodor or unpleasant taste of the untreated samples could be reduced.

Irrespective of the heating temperature, the samples that were concentrated in vacuum after treatment by indirect heating remained so malodorous or distasteful that they were not suitable for eating even after being given masking or flavoring treatment. The samples that were concentrated in vacuum after treatment by direct heating were substantially free of the malodorous or unpleasant taste imparting substances, and were ready to eat without further treatment.

These results suggest that the effectiveness of the method of the present invention is not due to the azeotropic distillation of the steam and malodorous or unpleasant taste imparting substances during concentration; rather, detachment of such undesired substances from the microbial cells that is caused by rapid blowing of pressurized steam seems responsible for the efficiency of the present invention. This effect was first discovered and applied in practice by the present inventors.

More than one cycle of treatment (heating and vacuum concentration) was conducted, but whether the heating was direct or indirect, the results were substantially the same as those obtained by performing only one cycle of treatment.

The same results were obtained when the test procedure described above was repeated for fermented wheys. With samples that were concentrated under vacuum after treatment by direct heating, the malodorous or unpleasant taste imparting substances were significantly reduced, and no separation or agglomeration occurred due to protein denaturation. The contents of vitamins (e.g. vitamins $B_1$ and $B_2$) decreased by only 2 - 4%. The flavor of the samples was so good that they were ready to drink without further treatment.

The test results are shown in the following table.

EP 0 147 517 B1

Table: Results of Treatment of Fermented 10% Solution of Whey Powder

-- Degree of reduction, as evaluated by organoleptic test, of malodorous or unpleasant taste imparting substances adhering to the cells of lactic-acid-producing bacteria after samples were first heated directly (by rapid blowing of pressurized steam) or indirectly (by passage through a plate-type heat exchanger), then immediately concentrating the samples at 1 199,5 - 332,5 mbar.--

| Fermentation starter *1) | Panel test *2) | Flavor of fermented whey *3) | Flavor of microbial cells *3) | Flavor after vacuum distillation *3) | | | | | |
|---|---|---|---|---|---|---|---|---|---|
| | | | | Preliminarily treated by rapid blowing of pressurized steam (parenthesized figures indicating the whey temperature) | | | Preliminarily treated by passage through plate-type heat exchanger | | |
| | | | | vapor pressure 1.5 kg/cm$^2$ (110°C) | vapor pressure 2.7 kg/cm$^2$ (130°C) | vapor pressure 4.7 kg/cm$^2$ (150°C) | whey temperature 110°C | whey temperature 130°C | whey temperature 150°C |
| (1) | 1 | D | D | B | A | A | C | C | C |
| | 2 | D | C | B | A | A | C | C | C |
| | 3 | D | D | B | B | A | D | D | C |
| | 4 | C | C | A | A | A | C | C | C |
| | 5 | D | C | A | A | A | C | C | C |
| | 6 | D. | C | B | A | A | C | C | C |
| | 7 | D | D | B | A | A | C | C | C |
| | Overall evaluation | D | C - D | A - B | A | A | C | C | C |

| | | (1) | (2) | (3) | (4) | (5) | (6) | (7) | (8) |
|---|---|---|---|---|---|---|---|---|---|
| (2) | 1 | C | D | D | A | B | B | D | D |
| | 2 | C | D | D | A | A | B | D | D |
| | 3 | D | D | D | B | C | C | D | D |
| | 4 | C | D | D | A | A | B | C | D |
| | 5 | C | D | D | A | B | B | D | D |
| | Overall evaluation | C | D | D | A | A – B | B | D | D |
| (3) | 1 | C | C | C | A | A | B | D | D |
| | 2 | C | C | C | A | A | B | D | D |
| | 3 | C | D | D | A | B | C | D | D |
| | 4 | C | C | C | A | A | A | C | D |
| | 5 | C | C | C | A | A | A | D | D |
| | Overall evaluation | C | C | C | A | A | A – B | D | D |

*1: Fermentation starters: (1) L. helveticus; (2) L. casei; (3) L. bulgaricus and Str. thermophilus (1:1).

*2: Panel test: (1) consisted of 7 healthy male adults having standard organoleptic preference, whereas (2) and (3) consisted of 5 such persons.

*3: Flavor of the fermented wheys samples and microbial cells:
A...Very slight malodor or unpleasant taste.
B...Some malodor or unpleasant taste, which was not sensible at all if masked with a flavor.
C...Some malodor or unpleasant taste remained even after masking with a flavor.
D...Malodor or unpleasant taste was so strong that it could not be masked with a flavor at all.

In the evaluation of flavor, a sweetener and yogurt flavors reminiscent of citrus fruits and vanilla were used as masking flavors. The yogurt flavors were of the type which are commonly used in yogurt manufacture to mask the unpleasant smell of fermented milk.

Example 1

Various lactic-acid-producing bacteria (each having 2 parts by weight) were inoculated as fermentation starters into culture mediums and fermentation was continued until the pH was 4.0. Before the experiment, the starters were sufficiently activated by culture in a medium consisting of a 10 wt% aqueous solution of skim milk powder. The mediums into which these starters were inoculated were prepared by first sterilizing 100 parts by weight of an aqueous solution containing 10 parts by weight of whey powder at 75°C for 15 seconds and then cooling the solution to specified fermentation temperatures.

The fermentation temperatures and periods varied with the starter bacteria as follows:

1) 42°C x 6 hr for L. helveticus;
2) 35°C x 20 hr for L. casei;
3) 45°C x 8 hr for L. bulgaricus/Str. thermophilus (1:1) mixture;
4) 45°C x 16 hr for Str. thermophilus; and
5) 37°C x 18 hr for Str. lactis.

All the fermented whey samples developed an extreme malodor or unpleasant taste and were hardly suitable for eating. This was particularly so with the solids portion separated from the fermented whey by high-speed centrifugation and which were largely composed of microbial cells. The solids portion was subsequently microfiltered, and since the filtrate had slightly malodor or unpleasant taste, it was concluded that, irrespective of the starter type, the malodorous or unpleasant taste imparting substances adhered to the microbial cells.

Samples of fermented whey or cell suspension (30°C) were continuously fed at one end into a Y-shaped, pressure-resistant stainless steel tube (ID: 7 mm) having valves preventing backflow. At another end, pressurized steam (2 - 7 kg/cm$^2$ 1961 - 6860 mbar) was fed so that the temperature of the fermentation liquor or cell suspension was immediately elevated to 110 - 150°C. The mixture flowing out at the third end of the tube was introduced in 5 seconds into a pressure-resistant stainless steel cylinder held at between 199,5 and 332,5 mbar, in which the mixture was concentrated to distill the malodorous or unpleasant taste imparting substances off the microbial cells. Due to the latent heat of evaporation, the temperatures of the concentrates immediately dropped to between 65 and 75°C, and they were further quenched in a cooler to 20°C.

All of the treated wheys thus obtained received good evaluation of flavor from the panelists (see Reference Example). They were substantially free from the denaturation of proteins (e.g. lactoalbumin and lactoglobulin) and the decomposition of vitamins.

Example 2

Whey that was protein-fortified by addition of 3 - 5 parts by weight of whey protein powder (80% protein) was used as a fermentation medium. The resulting fermented wheys were treated as in Example 1 and the products were found to be satisfactory in both flavor and nutritive value.

Example 3

Fermented wheys which were the same as used in Examples 1 and 2 were incorporated with 0.3 - 0.7 part by weight of pectin and deodorized as in Example 1 to provide acidic protein drinks which were satisfactory in both flavor and nutritive value. The drinks were free from protein denaturation for an extended period.

Example 4

Ten parts by weight of deproteinized whey (Permeate) powder was used as a medium for ferment on which was conducted as in Example 1. Because of the imbalance of nutrients in the medium, the fermented whey obtained had an extreme malodor and unpleasant taste. However, as a result of treatment by the method of the present invention, it was possible to significantly reduce the malodor or unpleasant taste and the concentrate was suitable for eating after masking with a flavor.

**Claims**

1.  A method of removing malodorous or unpleasant taste imparting substances from microbial cells comprising first blowing pressurized steam having a pressure of 1 - 10 kg/cm$^2$ (980.6 - 9806 mbar) and a temperature of 110 - 180°C into fermented whey containing said malodorous or unpleasant taste imparting substances, then introducing said whey into a vacuum atmosphere for concentrating and cooling said whey.

2.  A method according to Claim 1 wherein said pressurized steam has a pressure of 2 - 7 kg/cm$^2$ (1961 - 6860 mbar) and a temperature of 120 - 160°C.

3.  A method according to Claim 1 wherein said pressurized steam is blown into the fermented whey for a period of 1 - 20 seconds.

4.  A method according to Claim 3 wherein said pressurized steam is blown into the fermented whey for a period of 2 - 10 seconds.

5.  A method according to Claim 1 wherein said vacuum atmosphere has a pressure of 40 - 971 mbar.

6.  A method according to Claim 5 wherein said vacuum atmosphere has a pressure of 66,5 - 931 mbar.

7.  A method according to Claim 6 wherein said vacuum atmosphere has a pressure of 199,5 - 465,5 mbar.

8.  A method according to Claim 1 wherein the fermented whey containing malodorous or unpleasant taste imparting substances is whey that has been treated with a lactic-acid-producing bacterium.

9.  A method of removing malodorous or unpleasant taste imparting substances from fermented whey comprising blowing pressurized steam having a pressure of 1 - 10 kg/cm$^2$ (980.6 - 9806 mbar) and a temperature of 110 - 180°C for a period of 1 - 20 seconds into fermented whey which has been treated with a lactic-acid-producing bacterium, and introducing said fermented whey into a vacuum atmosphere having a pressure of 40 - 971 mbar for concentrating and cooling said whey.

10. A method according to Claim 9 wherein said fermented whey is fed with pressurized steam of a pressure of 2 - 7 kg/cm$^2$ (1961 - 6860 mbar) and a temperature of 120 - 160°C which is blown for a period of 2 - 10 seconds, followed by introducing said whey into a vacuum atmosphere having a pressure of 199,5 - 332,5 mbar.

**Revendications**

1.  Procédé pour éliminer des substances donnant de mauvaises odeurs ou de mauvais goûts de cellules microbiennes, comprenant tout d'abord l'insufflation de vapeur sous pression ayant une pression de 980,6 à 9 806 mbar (1 à 10 kg/cm$^2$) et une température de 110 à 180°C dans un lactosérum fermenté contenant lesdites substances donnant de mauvaises odeurs ou de mauvais goûts, puis l'introduction dudit lactosérum dans une atmosphère à dépression pour concentrer et refroidir ledit lactosérum.

2.  Procédé selon la revendication 1, dans lequel ladite vapeur sous pression a une pression de 1 961 à 6 860 mbar (2 à 7 kg/cm$^2$) et une température de 120 à 160°C.

3.  Procédé selon la revendication 1, dans lequel ladite vapeur sous pression est insufflée dans le lactosérum fermenté pendant une période de 1 à 20 secondes.

4.  Procédé selon la revendication 3, dans lequel ladite vapeur sous pression est insufflée dans le lactosérum fermenté pendant une période de 2 à 10 secondes.

5.  Procédé selon la revendication 1, dans lequel ladite atmosphère à dépression a une pression de 40 à 971 mbar.

**6.** Procédé selon la revendication 5, dans lequel ladite atmosphère à dépression a une pression de 66,5 à 931 mbar.

**7.** Procédé selon la revendication 6, dans lequel ladite atmosphère à dépression a une pression de 199,5 à 465,5 mbar.

**8.** Procédé selon la revendication 1, dans lequel le lactosérum fermenté contenant des substances donnant de mauvaises odeurs ou de mauvais goûts est un lactosérum que l'on a traité avec une bactérie productrice d'acide lactique.

**9.** Procédé pour éliminer des substances donnant de mauvaises odeurs ou de mauvais goûts d'un lactosérum fermenté, comprenant l'insufflation de vapeur sous pression, ayant une pression de 980,6 à 9 806 mbar (1 à 10 kg/cm²) et une température de 110 à 180°C, pendant une période de 1 à 20 secondes, dans un lactosérum fermenté que l'on a traité avec une bactérie productrice d'acide lactique, et l'introduction dudit lactosérum fermenté dans une atmosphère à dépression ayant une pression de 40 à 971 mbar pour concentrer et refroidir ledit lactosérum.

**10.** Procédé selon la revendication 9, dans lequel on introduit dans ledit lactosérum fermenté de la vapeur sous pression ayant une pression de 1 961 à 6 860 mbar (2 à 7 kg/cm²) et une température de 120 à 160°C, que l'on insuffle pendant une période de 2 à 10 secondes, ledit lactosérum étant ensuite introduit dans une atmosphère à dépression ayant une pression de 199,5 à 332,5 mbar.

## Patentansprüche

**1.** Verfahren zur Entfernung von übelriechenden oder einen unangenehmen Geschmack verleihenden Substanzen aus mikrobiellen Zellen, umfassend zunächst das Einblasen von unter Druck stehendem Dampf mit einem Druck von 1 bis 10 kg/cm² (980,6 bis 9806 mbar) und einer Temperatur von 110 bis 180°C in fermentierte Molke, die diese übelriechenden oder einen unangenehmen Geschmack verleihenden Substanzen enthält, dann das Einbringen der Molke in eine Vakuumatmosphäre zum Konzentrieren und Abkühlen der Molke.

**2.** Verfahren nach Anspruch 1, wobei der unter Druck stehende Dampf einen Druck von 2 bis 7 kg/cm² (1961-6860 mbar) und eine Temperatur von 120 bis 160°C aufweist.

**3.** Verfahren nach Anspruch 1, wobei der unter Druck stehende Dampf in einem Zeitraum von 1 bis 20 Sekunden in die fermentierte Molke eingeblasen wird.

**4.** Verfahren nach Anspruch 3, wobei der unter Druck stehende Dampf in einem Zeitraum von 2 bis 10 Sekunden in die fermentierte Molke eingeblasen wird.

**5.** Verfahren nach Anspruch 1, wobei die Vakuumatmosphäre einen Druck von 40 bis 971 mbar aufweist.

**6.** Verfahren nach Anspruch 5, wobei die Vakuumatmosphäre einen Druck von 66,5 bis 931 mbar aufweist.

**7.** Verfahren nach Anspruch 6, wobei die Vakuumatmosphäre einen Druck von 199,5 bis 465,5 mbar aufweist.

**8.** Verfahren nach Anspruch 1, wobei die fermentierte Molke, die die übelriechenden oder einen unangenehmen Geschmack verleihenden Substanzen enthält, Molke ist, die mit einem Milchsäure produzierenden Bakterium behandelt worden ist.

**9.** Verfahren zur Entfernung von übelriechenden oder einen unangenehmen Geschmack verleihenden Substanzen aus fermentierter Molke, umfassend das Einblasen von unter Druck stehendem Dampf mit einem Druck von 1 bis 10 kg/cm² (980,6 bis 9806 mbar) und einer Temperatur von 110 bis 180°C in einem Zeitraum von 1 bis 20 Sekunden in fermentierte Molke, die mit einem Milchsäure produzierenden Bakterium behandelt worden ist, und Einbringen der fermentierten Molke in eine Vakuumatmosphäre mit einem Druck von 40 bis 971 mbar zum Konzentrieren und Abkühlen der Molke.

**10.** Verfahren nach Anspruch 9, wobei der fermentierten Molke unter Druck stehender Dampf mit einem Druck von 2 bis 7 kg/cm$^2$ (1961-6860 mbar) und einer Temperatur von 120 bis 160°C zugeführt wird, der in einem Zeitraum von 2 bis 10 Sekunden eingeblasen wird, gefolgt vom Einbringen der Molke in eine Vakuumatmosphäre mit einem Druck von 199,5 - 332,5 mbar.